(19) Europäisches Patentamt
European Patent Office
Office européen des brevets

(11) Veröffentlichungsnummer: **0 098 367**
**A2**

(12) # EUROPÄISCHE PATENTANMELDUNG

(21) Anmeldenummer: **83104735.2**

(22) Anmeldetag: **13.05.83**

(51) Int. Cl.³: **C 07 D 313/00**

(30) Priorität: **02.07.82 DE 3224707**

(43) Veröffentlichungstag der Anmeldung: **18.01.84**
**Patentblatt 84/3**

(84) Benannte Vertragsstaaten: **AT CH DE FR GB LI NL**

(71) Anmelder: **CHEMISCHE WERKE HÜLS AG, - RSP Patente / PB 15 - Postfach 13 20, D-4370 Marl 1 (DE)**

(72) Erfinder: **Bartmann, Martin, Dr., Burgstrasse 35, D-4350 Recklinghausen (DE)**
Erfinder: **Burzin, Klaus, Dr., Wellerfeldweg 164, D-4370 Marl (DE)**

(54) **Verfahren zur Herstellung von makrocyclischen Ketolactonen.**

(57) Die Erfindung betrifft ein Verfahren zur Herstellung makrocyclischer Ketolactone der Formel

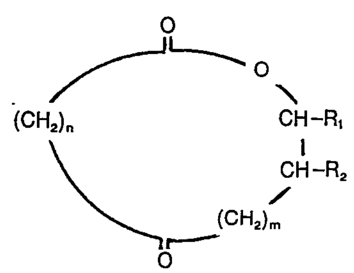

I

mit $n = 6$ bis 12, $m = 0$ bis 3 und $R_1$ und $R_2$ = H, $C_1$–$C_4$-Alkyl, wobei die entsprechenden bicyclischen Enolether mit einem Gemisch aus wäßrigem Wasserstoffperoxid und Ameisensäure behandelt werden.

EP 0 098 367 A2

## Verfahren zur Herstellung von makrocyclischen Ketolactonen

Makrocyclische Ketolactone der Formel

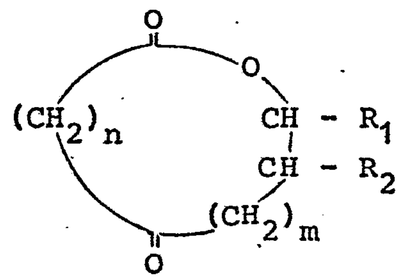

, I

wobei n = 6 bis 12 und m = 0 bis 3 und $R_1$ und $R_2$ unabhängig voneinander Wasserstoff oder ein Alkylrest mit 1 bis 4 C-Atomen, insbesondere ein Methylrest, bedeuten, sind als intensive und angenehm duftende Moschusriechstoffe grundsätzlich bekannt, vgl. Ohloff, Fortschritte der chemischen Forschung 12, 203 (1969).

Nach der DE-OS 24 10 859 wurden Vertreter von I mit n = 9 bis 13 und m = 0 bis 2 sowie $R_1$, $R_2$ = H, $-CH_3$, $-CH_2CH_3$ durch Umsetzung der bicyclischen Enolether

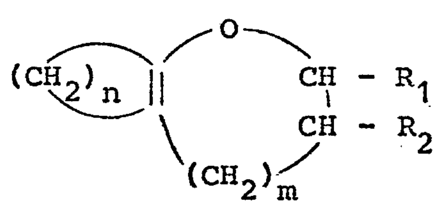

II

mit Ozon hergestellt. Nach I. J. Borowitz et al. (J. org. Chem. 31, 3032 (1966) und 37, 581 (1972) erhält man die Ketolactone I mit n = 4 bis 6 und m = 1,2 und $R_1$, $R_2$ = H aus den Verbindungen II auch durch Umsetzung mit Derivaten der Perbenzoesäure.

Diese beiden Verfahren sind in mehrfacher Hinsicht unbefriedigend.

BAD ORIGINAL

1. Ozon ist mit einem MAK-Wert von 0,2 $mg/m^3$ ein außerordentlich toxisches Gas. Eine Apparatur im technischen Maßstab ist daher aufwendig, die Durchführung eines solchen Verfahrens ist nicht unproblematisch.

2. Die komplexe Ozonolyse wirft Aufarbeitungsprobleme auf (vgl. Borowitz et al., J. Org. Chem. 31, 3032 (1966).

3. Setzt man Perbenzoesäure oder ihre Derivate zur Oxidation ein, so müssen diese aromatischen Persäuren zunächst einmal aus Wasserstoffperoxid und Benzoesäure hergestellt werden. Die Ausbeuten hierfür liegen bei 80 %.

4. Die Abtrennung der in Wasser unlöslichen Benzoe- und/oder Perbenzoesäure erfordert mehrere Reinigungsschritte.

5. Die Ausbeuten an Ketolactonen sind - abgesehen von dem unsubstituierten 6-Oxo-nonanolid ($n = 4$, $m = 1$, $R_1$, $R_2 = H$) - unbefriedigend.

Aufgabe dieser Erfindung war es daher, ein verbessertes Verfahren zur Herstellung der Ketolactone I mit $n = 6$ bis 12, $m = 0$ bis 3 und $R_1$, $R_2$ = H, $C_1$-$C_4$-Alkyl zu entwickeln.

Es wurde jetzt gefunden, daß sich die bicyclischen Enolether der Formel II in guter Ausbeute mittels wäßriger Wasserstoffperoxid-/Ameisensäure-Lösungen zu den Ketolactonen I oxidativ öffnen lassen. Das Verfahren läßt sich, wie aus dem experimentellen Teil ersichtlich ist, problemlos durchführen. Es sind insbesondere keine zeitraubenden Trennoperationen erforderlich.

Der gefundene Reaktionsweg ist für den Fachmann überraschend. Nach Borowitz et al. erhält man nämlich bei der Umsetzung von bicyclischen Enolethern mit feuchter Persäure nicht Ketolactone, sondern Glykole der Struktur

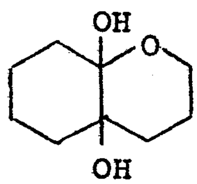 ,

die dann erst mit Bleitetraacetat zu den Ketolactonen I umgesetzt werden müssen.

Ein weiteres Verfahren der Oxidation mit organischen Persäuren wird in der DE-PS 20 65 550 beschrieben (vgl. auch DE-AS 20 26 056). Danach wird eine Lösung von 13-Oxabicyclo-(10.4.0)-hexadecen-(1) in 90 %iger Essigsäure mit einer Mischung aus 52 %igem Wasserstoffperoxid und konzentrierter Schwefelsäure behandelt.

Das erhaltene Hydroperoxidaddukt wird in siedendes Xylol eingetragen und ergibt ein Gemisch aus Pentadecanolid, Pentadecen-11-olid sowie 12-Hydroxy-pentadecanolid, nicht jedoch ein cyclisches Ketolacton analog zu Formel I.

Für das Verfahren gemäß der hier vorliegenden Erfindung ist es entscheidend, daß man eine Mischung aus Ameisensäure und wäßrigem Wasserstoffperoxid einsetzt.

Das Verfahren wird in erster Linie bei bicyclischen Enolethern eingesetzt, wobei n = 6 bis 12, m = 0 bis 3 und $R_1$ und $R_2$ unabhängig voneinander Wasserstoff oder ein Alkylrest mit 1 bis 4 C-Atomen, vorzugsweise n = 6 bis 10 und $R_1$ und $R_2$ unabhängig voneinander Wasserstoff oder einen Methylrest bedeuten. Das Verfahren ist nicht auf bicyclische Enolether beschränkt: vielmehr eigenet es sich grundsätzlich zur Oxidation elektronenreicher Olefine.

Die oxidative Öffnung der bicyclischen Enolether II erfolgt zweckmäßigerweise, indem man diese in einem Lösemittel auflöst und mit einer Mischung aus wäßrigem Wasserstoffperoxid und Ameisensäure versetzt und bei einer Temperatur zwischen -20 °C und +120 °C zur Reaktion bringt.

Als Lösungsmittel eignen sich besonders 1,2-Dichlorethan, Dichlormethan, Trichlormethan, Chlorbenzol sowie Essigsäure und Ameisensäure als auch Gemische aus diesen Substanzen. Die Reaktionstemperaturen liegen im allgemeinen zwischen 20 und 80 °C, bevorzugt zwischen 40 und 70 °C. Nach beendeter Reaktion (1 bis 4 Stunden) trennt man die wäßrige Phase ab, wäscht die organische Phase säurefrei und fraktioniert anschließend den getrockneten Rückstand.

Die als Edukte dienenden bicyclischen Enolether, wie z. B. 12-Oxa-bicyclo(9,3,0)tetradecen-(1), 13-Oxa-bicyclo-(10,4,0)hexadecen-(1) und das 14-Oxa-bicyclo(11,4,0)-heptadecen-(1) sind bekannt und lassen sich nach bekannten Verfahren herstellen (DE-OS 21 36 496, DE-OS 20 26 056).

Dementsprechend seien als Ketolactone, welche nach dem erfindungsgemäßen Verfahren hergestellt werden können, beispielsweise genannt: 11-Oxo-tridecanolid, 12-Oxo-pentadecanolid, 13-Oxo-hexadecanolid.

Die erhaltenen Ketolactone lassen sich nach bekannten Methoden in die parfümistisch sehr wertvollen unstabilisierten makrocyclischen Lactone, wie z. B. Exaltolid, umwandeln (DE-OS 27 31 543, J. org. Chem. USSR <u>15</u>, 1285 (1979).

Für Riechstoffkompositionen sind die nach dem erfindungsgemäßen Verfahren hergestellten Ketolactone als Moschuskomponenten und Fixateure einsetzbar (DE-OS 24 10 859).

## Beispiel 1

44 g (0,2 Mol) 13-Oxa-bicyclo(10,4,0)hexadecen-(1) wurden in 150 ml Chloroform gelöst. Die Mischung wurde unter Rühren und Stickstoffabdeckung zum Sieden erhitzt. Anschließend tropfte man eine Mischung aus 14 g (0,3 Mol) Ameisensäure und 34,8 g (0,6 Mol) $H_2O_2$ (als 60 %ige wäßrige Lösung) ohne Heizung so schnell hinzu, daß der Rückfluß erhalten blieb. Nach beendeter Zugabe ließ man noch 3 Stunden bei Siedetemperatur nachreagieren, wusch 2mal mit Wasser, 2mal mit wäßriger Natriumhydrogencarbonatlösung und nochmals mit Wasser, trocknete die organische Phase und destillierte 12-Oxo-pentadecanolid ab.

| | |
|---|---|
| Ausbeute: | 85 % |
| Siedepunkt: | 145 bis 150 °C (0,1 mbar) |
| Schmelzpunkt (PE): | 32 °C ± 1 |
| IR: | 1 760 $cm^{-1}$ + 1 720 $cm^{-1}$ |
| NMR ($CDCl_3$): | 4,1 δ (Triplett, $-CH_2-O-CO-$, 2 H) |
| Geruch: | nach Moschuskörnern |

## Beispiel 2

45 g (0,2 Mol) 15-Methyl-13-oxa-bicyclo(10,4,0)hexadecen-(1) wurden entsprechend Beispiel 1 mit $H_2O_2$/Ameisensäure zum 12-Oxo-14-methyl-pentadecanolid umgesetzt.

| | |
|---|---|
| Ausbeute: | 82 % |
| Siedepunkt: | 148 bis 153 °C (0,1 mbar) |
| IR: | 1 755 $cm^{-1}$ + 1 720 $cm^{-1}$ |
| NMR: | 4,1 δ (Triplett, $-CH_2-O-CO-$, 2 H) |
| | 1,2 δ (Dublett, $\underline{CH_3}-CH$, 3 H) |

Beispiel 3

41 g (0,2 Mol) 10-Methyl-9-oxa-bicyclo(6,5,0)tridecen-(1) wurden entsprechend Beispiel 1 mit $H_2O_2$/Ameisensäure zum 8-Oxo-12-methyl-dodecanolid umgesetzt.

| | |
|---|---|
| Ausbeute: | 91 % |
| Siedepunkt: | 133 bis 136 °C (0,7 mbar) |
| IR: | 1 760 $cm^{-1}$ + 1 720 $cm^{-1}$ |
| NMR: | 4,4 δ (Multiplett, $-\underset{\mid \atop CH_3}{\underline{CH}}-O-CO-$, 1 H) |

Patentansprüche:

1. Verfahren zur Herstellung von makrocyclischen Ketolactonen der Formel

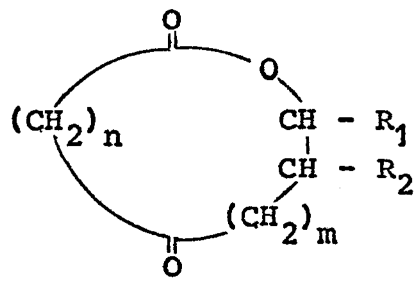
, I

worin n = 6 bis 12, m = 0 bis 3 und $R_1$ und $R_2$ unabhängig voneinander Wasserstoff oder ein Alkylrest mit 1 bis 4 C-Atomen bedeuten, durch oxidative Ringöffnung von bicyclischen Enolethern der Formel

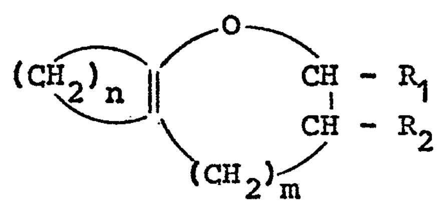

dadurch gekennzeichnet,
daß man die bicyclischen Enolether mit einem Gemisch aus wäßrigem Wasserstoffperoxid und Ameisensäure behandelt.

2. Verfahren nach Anspruch 1,
dadurch gekennzeichnet,
daß man Ketolactone einsetzt, worin n = 6 bis 10 und $R_1$ und $R_2$ unabhängig voneinander Wasserstoff oder ein Methylrest bedeuten.

3. Verfahren nach den Ansprüchen 1 oder 2,
dadurch gekennzeichnet,
daß man eine 30 bis 80 gewichtsprozentige wäßrige Wasserstoffperoxidlösung einsetzt.